# EUROPEAN PATENT APPLICATION

(11) **EP 1 839 623 A1**
(43) Date of publication of application: **03.10.2007**
(21) Application number: 07006302.9
(22) Date of filing: 27.03.2007
(51) Int. Cl.: A61F 2/06

(54) **Stent graft with healing promoting necks**

(30) Priority: 28.03.2006 US 277643
(71) Applicant: Medtronic Vascular, Inc., Santa Rosa, California 95403 (US)
(72) Inventor: Hendriks, Marc, 6441 KD Brunssum (NL); Koullick, Edouard, South Golden Valley, Minnesota 55416 (US); Elkins, Jeff, Woodside, California 94062 (US); Billy, Didier, 5229 VW Maastricht (NL); Kwitkin, Brian, Pembroke Pines, Florida 33028 (US); Van Bilsen, Paul, 6224 HV Maastricht (NL); Chu, Jack, Santa Rosa, California 95409 (US); Raze, Brian, Ham Lake, Minnesota 55304 (US)
(74) Representative: Zimmermann, Gerd Heinrich

(57) **Abstract**

An endoluminal stent graft (100) comprises a stent structure; a graft material (106) attached to said stent structure and having a circumferential edge (122, 124); an anchor region (102, 104) extending longitudinally along said graft material (106) from said circumferential edge (122, 124); and a healing promoter (116A, 116B) located within said anchor region (102, 104) wherein said healing promoter (116A, 116B) aids in anchoring said endoluminal stent graft (100) in a vessel, wherein said healing promoter (116A, 116B) includes a first portion on an exterior circumferential surface of said graft material.

## Description

### BACKGROUND OF THE INVENTION

### FIELD OF THE INVENTION

The present invention relates generally to stent grafts, and more particularly to improving healing associated with placement of an endoluminal stent graft in a vessel.

### DESCRIPTION OF THE RELATED ART

Vascular aneurysms are the result of abnormal dilation of a blood vessel, usually resulting from disease and/or genetic predisposition, which can weaken the arterial wall and allow the wall to expand. While aneurysms can occur in any blood vessel, most occur in the aorta and peripheral arteries, with the majority of aortic aneurysms occurring in the abdominal aorta, usually beginning below the renal arteries and often extending distally into one or both of the iliac arteries.

Aortic aneurysms are often treated in open surgical procedures where the diseased vessel segment is bypassed and repaired with an artificial vascular graft. While considered an effective surgical technique, conventional vascular graft surgery however, is frequently not advisable for elderly patients or those patients weakened from cardiovascular and/or other diseases.

An alternative treatment to the open surgical procedure is placement of an endovascular prosthesis, such as an endoluminal stent graft, inside the vessel to isolate the aneurysm from blood flow and subsequent pressure. Generally, endoluminal stent grafts are delivered to a desired location within a vessel using a catheter-based delivery technique. To deliver the endoluminal stent graft within an acceptable size for a blood vessel, endoluminal stent grafts are typically compressed and housed in a removable sheathing. The endoluminal stent graft is then inserted into a vessel via the catheter-based delivery technique, positioned in the vessel, and the sheath removed allowing the endoluminal stent graft to expand and contact the vessel walls.

Endoluminal stent grafts typically include a graft material supported by a stent structure. Generally, endoluminal stent grafts are formed in a tubular shape with proximal and distal neck openings to allow for blood flow. Conventionally, the proximal end of the endoluminal stent graft is referenced with respect to the end closest to the heart (via the length of blood traveled from the heart.) Some endoluminal stent grafts further include openings or bifurcations to accommodate lateral branches off the main vessel.

Implantation of endoluminal stent grafts in the prior art can be subject to a number of technical problems with subsequent morbidity and mortality. In some patients, the proximal neck of the prior art endoluminal stent grafts do not heal in well to the vessel wall. The lack of healing in and incorporation of the endoluminal stent graft at the aneurysm neck can allow the endoluminal stent graft to dislodge and migrate distally inside the aortic vessel permitting renewed feeding of blood and pressure to the aneurysm sac with the consequent risk of aneurysm rupture. Markedly affected were patients with severe neck angularity, and those with an aortic neck shorter than 10mm, due to insufficient contact surface with the vessel and insufficient anchoring force associated with the short neck.

Therefore, it is an object of the present invention to provide an endoluminal stent graft which, at least partially, overcomes the above described problems associated with the prior art.

### SUMMARY OF THE INVENTION

This object is solved by an endoluminal stent graft according to claim 1. Further aspects, features, details and advantages of the present invention are apparent from the dependent claims, the specification, and the accompanying drawings.

According to an embodiment of the present invention, an endoluminal stent graft comprises a stent structure, a graft material attached to said stent structure and having a circumferential edge, an anchor region extending longitudinally along said graft material from said circumferential edge, and a healing promoter located within said anchor region wherein said healing promoter aids in anchoring said endoluminal stent graft in a vessel, wherein said healing promoter includes a first portion on an exterior circumferential surface of said graft material. When correctly positioned within a vessel, the healing promoting material promotes cellular in growth and healing in of the vessel wall to the endoluminal stent graft, thus reducing the risk of distal migration and the occurrence of endoleaks that could otherwise form at the sides of the distal and proximal necks and the consequent feeding of the aneurysm sac. According to further embodiments, the healing promoting material is located at the proximal neck and/or the distal neck of the endoluminal stent graft. In still another embodiment, a ring-like insert in included in the endoluminal stent graft and optionally covered with a material that promotes healing.

### BRIEF DESCRIPTION OF THE DRAWINGS

FIG. 1 illustrates one example of an endoluminal stent graft including a healing promoter located within a distal anchor region and a proximal anchor region;

FIG. 2 illustrates one example of the healing promoter including a loop-like structure and a tail like structure;

FIG. 3 illustrates one example of an endoluminal stent graft including the healing promoter located within a distal anchor region and a proximal anchor region in accordance on both interior and exterior surfaces of the endoluminal stent graft;

FIG. 4 illustrates one example of an endoluminal stent graft including the healing promoter that, in turn, includes a ring-like insert;

FIG. 5 illustrates another example of an endoluminal stent graft including the healing promoter located within a proximal anchor region;

FIG. 6 illustrates yet another example of an endoluminal stent graft including the healing promoter located within a proximal anchor region;

FIG. 7 illustrates still yet another example of an endoluminal stent graft including the healing promoter located within a proximal anchor region; and

FIG. 8 illustrates one example of an endoluminal stent graft including the healing promoter located within a proximal anchor region and a distal anchor region.

Common reference numerals are used throughout the drawings and detailed description to indicate like elements.

### DETAILED DESCRIPTION

FIG. I illustrates one example of an endoluminal stent graft 100 including a healing promoter 116A, 116B located at selected positions on a graft material 106. Endoluminal stent graft 100, herein termed simply stent graft 100, includes: a graft material 106, i.e., a first material; healing promoter 116A, 116B positioned about an exterior circumferential surface of the first material, and a stent structure of shaped springs, such as a first (base) spring 110, a second (support) spring 112, and an anchor spring 114, among others, distributed within stent graft 100 and attached to graft material 106. Stent graft 100 is shaped to form a lumen 108 that bifurcates distally to accommodate lateral vessels, e.g., the common iliac arteries. Optionally, an extension 120 is included as part of stent graft 100 for some applications.

In one example, graft material 106 is a material formed to limit the leakage of blood through graft material 106. Examples of graft material 106 include substantially non-porous fabrics, such as low profile system (LPS) material, or densely knitted fabrics. Any of the commonly used graft materials are suitable for use herein.

As illustrated, proximal anchor region 102 is located at a proximal neck of stent graft 100, and healing promoter 116A forms a right circular cylinder around stent graft 100 within proximal anchor region 102 on an exterior circumferential surface of graft material 106. In this example, proximal anchor region 102 extends longitudinally from a proximal circumferential edge 122 longitudinally toward the distal end of stent graft 100 a specified distance W_proximal along an outer circumferential surface of stent graft 100. W_proximal should be in contact with tissue (endothelium inner layer of the vessel). Therefore, W-proximal should be, ideally, a distance equal to the aneurysm neck (AAA). This distance is usually determined in the individual patient by echography (ultrasonography) or Computed Tomography imaging (CT scanning, CT Scan). In one example, specified distance W_proximal defines a length of what is commonly referred to as the proximal neck of stent graft 100. Thus, a group of stent grafts is provided having a range of specified distances W_proximal so that the range of specified distances corresponds to the range of aneurysm necks commonly encountered in patients. A physician chooses a particular stent graft in the group based on the characteristics of the aneurysm neck in a particular patient.

Distal anchor region 104 is located at a distal neck of leg 118 of stent graft 100, and healing promoter 116B is attached to leg 118 within a distal anchor region 104 on an exterior circumferential surface of graft material 106 of leg 118. In this example, distal anchor region 104 extends from a distal circumferential edge 124 of leg 118 a specified longitudinal distance W _distal towards the proximal end of stent graft 100 and along an outer circumferential surface of leg 118. In the distal part of the graft the presumption is that the graft is substantially in contact with the inner endothelium tissue of the iliac artery. If this is indeed the case, then W-distance is chosen to be in the range of 5-10mm. In one example, specified distance W_distal defines a length of what is commonly referred to as the distal neck of leg 118 of stent graft 100.

In one embodiment, healing promoter 116A, 116B is a substance that supports cellular in growth that aids in fixation of an endoluminal stent graft, such as stent graft 100, within a vessel. Location of healing promoter 116A in proximal anchor region 102 and promoter 116B in distal anchor region 104 promotes healing in of the proximal and distal necks, respectively, of stent graft 100 in a vessel reducing the risk of dislodgement and migration, thus reducing the occurrence of endoleaks that could otherwise form at the proximal and distal proximal necks.

In one example, healing promoter 116 is a porous fabric, such as a Dacron fabric, or non-woven material. Healing promoter should be a material with a "non-smooth surface". In the particular case of AAA-endovascular graft, mainly endothelial, fibroblast and smooth muscle cells are able to adhere and migrate on the added "healing promoter" exposed surface. Cellular adhesion potential is related to the degree of roughness and wettability/surface charge (i.e. hydrophobicity) of the material surface (e.g. polymeric materials with a smooth surface inducing low cellular adhesion, demonstrate significant increased adhesion strength associated with increased surface roughness). In this respect, "knitted Dacron" (PET: polyethylene terephthalate) material would be appropriate. Fibrous polyurethane material could also be used. Porosity usually concerns material with a 3-D structure. However, certain non-woven PET fabrics (2-D) present defined porosity surfaces (low and high-porosity matrix). Pore sizes of at least 5µm would be appropriate to stimulate tissue in growth, with pore sizes of at least 50µm being more appropriate, and pore sizes of at least 100µm being most appropriate.

In one example, healing promoter 116A, 116B includes a coating on a material that further promotes healing-in, such as a collagen coating. Collagen or any other peptide, protein or free amine group containing healing-promoting biomolecule can be coated onto the stent graft through a two-step process. The first step comprises grafting of an acrylic acid/acryl amide copolymer onto the Dacron substrate, after which collagen is immobilized onto the available functional groups in the acrylic acid/acrylamide copolymer graft.

**Experimental Procedure:**

Graft procedure;

Two different types of stent graft materials, high density, e.g., as has been provided with the AneuRx^{®} stent graft products, are grafted with acrylic acid/acryl amide. For grafting, an aqueous solution containing 25 wt% acrylic acid and 5 wt% acryl amide monomer is used. Grafting is performed for 30 minutes followed by overnight rinsing to stop the grafting process. Small pieces of each material are stained with Toluidine Blue (TB), whereby blue staining denotes successful copolymer grafting. When compared to also stained reference materials, the acrylic acid / acryl amide graft appeared clearly present on both the high density and RPM stent graft materials through the observed uptake of the dye. The intensity of the blue stain was the same on either substrate.

Next, both stent graft materials were immobilized with collagen;

Collagen immobilization;

One part of each type of material was immersed in a MES buffer containing hydroxysuccinimide and N-(3-dimethylaminopropyl)-N'-ethylcarbodiimide hydrochloride (EDC). The reaction was allowed to continue for 30 minutes only at rT. After a quick rinse in deionized water the samples were immersed in a 0.5 wt% collagen solution in MES buffer for 16-18 hours at rT. Subsequently the samples were rinsed and dried at ambient conditions.

Staining with Coomassie Blue (protein dye) after collagen immobilization confirmed the presence of immobilized collagen on both stent graft materials. Collagen presence was also confirmed with FT-IR spectroscopy and X-Ray photonelectron spectroscopy.

In another example, healing promoter 116A, 116B includes at least one growth factor promoting agent, such as a ReGeneraTing Agent (RGTA). RGTA is chemically substituted dextran. RGTA is encapsulated in a material forming healing promoter 116A, 116B, or alternatively is applied directly to the material forming healing promoter 116A, 116B, for example, as a coating.

RGTA can be coated onto the stent graft as per the following procedure. First, RGTA will undergo controlled periodate oxidation as per the procedure previously described in US Patent 5,679,659 assigned to Medtronic, Inc. The periodate oxidized RGTA can then be immobilized onto the Dacron stent graft as follows: The Dacron stent graft material is provided with an acrylic acid/acrylamide copolymer graft as previously described [see previous paragraph 0026]. In a multi-step process that is similar to the procedure previously described in US Patent 5,607,475 assigned to Medtronic, Inc., then the periodate oxidized RGTA is immobilized and coated onto the stent graft material.

One could also use different approaches, using direct coating of collagen such as described by van der Bas et al. J Vasc Surg 2002. Instead of van der Bas' proposed impregnation of the collagen coating with growth factors, we could have the collagen also impregnated with RGTA, or any other desirable bioactive molecule.

Also, the Dacron substrate can be coated with a polymer overcoat, into which 'releaseable' bioactive substances, such as RGTA, can be imbibed. (Basically, the drug eluting stent approach). Many polymers could be used as a potential coating, e.g., synthetic, natural, biodegradable.

Synthetic polymers include alkyl cellulose, cellulose esters, cellulose ethers, hydroxyalkyl celluloses, nitrocelluloses, polyalkylene glycols, polyalkylene oxides, polyalkylene terephthalates, polyalkylenes, polyamides, polyanhydrides, polycarbonates, polyesters, polyglycolides, polymers of acrylic and methacrylic esters, polyacrylamides, polyorthoesters, polyphosphazenes, polysiloxanes, polyurethanes, polyvinyl alcohols, polyvinyl esters, polyvinyl ethers, polyvinyl halides, polyvinylpyrrolidone, poly(ether ether ketone)s, silicone-based polymers and blends and copolymers of the above. Specific examples of these broad classes of polymers include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), poly(vinyl acetate), poly(vinyl chloride), polystyrene, polyurethane, poly(lactic acid), poly(butyric acid), poly(valeric acid), poly[lactide-co-glycolide], poly(fumaric acid), poly(maleic acid), copolymers of poly (caprolactone) or poly (lactic acid) with polyethylene glycol and blends thereof.

Coatings may be non-biodegradable. Examples of preferred non-biodegradable polymers include ethylene vinyl acetate (EVA), poly(meth)acrylic acid, polyamides, silicone-based polymers and copolymers and mixtures thereof.

Coatings may be biodegradable. The rate of degradation of the biodegradable coating is determined by factors such as configurational structure, copolymer ratio, crystallinity, molecular weight, morphology, stresses, amount of residual monomer, porosity and site of implantation. Examples of biodegradable polymers include synthetic polymers such as polyesters, polyanhydrides, poly(ortho)esters, polyurethanes, siloxane-based polyurethanes, poly(butyric acid), tyrosine-based polycarbonates, and natural polymers and polymers derived therefrom such as albumin, alginate, casein, chitin, chitosan, collagen, dextran, elastin, proteoglycans, gelatin and other hydrophilic proteins, glutin, zein and other prolamines and hydrophobic proteins, starch and other polysaccharides including cellulose and derivatives thereof (e.g. methyl cellulose, ethyl cellulose, hydroxypropyl cellulose, hydroxy-propyl methyl cellulose, hydroxybutyl methyl cellulose, carboxymethyl cellulose, cellulose acetate, cellulose propionate, cellulose acetate butyrate, cellulose acetate phthalate, cellulose acetate succinate, hydroxypropylmethylcellulose phthalate, cellulose triacetate, cellulose sulphate), poly-l-lysine, polyethylenimine, poly(allyl amine), polyhyaluronic acids, and combinations, copolymers, mixtures and chemical derivatives thereof (substitutions, additions of chemical groups, for example, alkyl, alkylene, hydroxylations, oxidations, and other modifications routinely made by those skilled in the art). In general, these materials degrade either by enzymatic hydrolysis or exposure to water in vivo, by surface or bulk erosion. The foregoing materials may be used alone, as physical mixtures (blends), or as a co-polymer. Biodegradable polyesters are for instance poly(glycolic acid) (PGA), poly(lactic acid) (PLA), poly(glycolic-co-lactic acid) (PGLA), poly(dioxanone), poly(caprolactone) (PCL), poly(3-hydroxybutyrate) (PHB), poly(3-hydroxyvalerate) (PHV), poly(lactide-co-caprolactone) (PLCL), poly(valerolactone) (PVL), poly(tartronic acid), poly(b-malonic acid), poly(propylene fumarate) (PPF) (preferably photo cross-linkable), poly(ethylene glycol)/poly(lactic acid) (PELA) block copolymer, poly(L-lactic acid-e-caprolactone) copolymer, and poly(lactide)-poly(ethylene glycol) copolymers. Biodegradable polyanhydrides are for instance poly[1,6-bis(carboxyphenoxy)hexane], poly(fumaric-co-sebacic)acid or P(FA:SA), and such polyanhydrides may be used in the form of copolymers with polyimides or poly(anhydrides-co-imides) such as poly-[trimellitylimidoglycine-co-bis(carboxyphenoxy)hexane], poly[pyromellitylimidoalanine-co-1,6-bis(carboph-enoxy)-hexane], poly[sebacic acid-co-1,6-bis(p-carboxyphenoxy)hexane] or P(SA:CPH) and poly[sebacic acid□co-1,3-bis(p-carboxyphenoxy)propane] or P(SA:CPP).

It has been shown that about 20 µg RGTA in decellularized tissue could induce good healing process.["A synthetic glycosaminoglycan mimetic binds vascular endothelial growth factor and modulates angiogenesis" Vincent Rouet et al. J Biol Chem. 2005 Jul 13]. Rather than sue RGTA alone as a coating, we would combine RGTA with another material, not just coat it as is. Either covalent immobilization as described in more detail above, or imbibement / impregnation with a natural or synthetic (biodegradable) polymer overcoat.

Obviously, we can make the coating 'exotic' - a first overcoat capable of releasing RGTA, followed by a second overcoat consisting of collagen, though such a process might be considered to be not practical.

Alternatively, rather than using a material, healing promoter 116A, 116B is a coating directly on graft material 106. In one example, healing promoter 116A, 116B is a drug impregnated coating that promotes the formation of thrombosis and tissue incorporation between stent graft 100 and a vessel. In another example, healing promoter 116A, 116B is coated on graft material 106, portions of the stent structure, such as any of first base spring 110, second support spring 112, and anchor spring 114, among others, or both.

To follow the copolymer grafting procedure as further detailed above, for metal substrates we would need a silanization priming step, similar to the one described in US Patent 5,607,475 assigned to Medtronic, Inc. Alternatively, for metal substrates, when wanting to use polymer based drug eluting coatings, first primer layers might be needed such as those well known and used for drug eluting stents. In case the stent graft material is not Dacron, but ePTFE, another widely used graft material, then also the ePTFE may need to be pre-treated prior to either copolymer grafting or applying an overcoat. Suited pre-treatment methods can be found in the vacuum deposition or irradiation technologies [e.g., L. J. Matienzo, J. A. Zimmerman, and F. D. Egitto, Surface modification of fluoropolymers with vacuum ultraviolet irradiation, Journal of Vacuum Science & Technology A: Vacuum, Surfaces, and Films, Volume 12, Issue 5, pp. 2662-2671, 1994; M. K. Shi, L. Martinu, E. Sacher, A. Selmani, M. R. Wertheimer, A. Yelon, Angle-resolved XPS study of plasma-treated teflon PFA surfaces, Surface and Interface Analysis, Volume 23, Issue 2 , Pages 99 - 104, 1995]; moreover wet chemical modification of ePTFE (Teflon) has been described comprising reduction of the carbon-fluorine bonds with the purpose of modifying its adhesive and wetting surface properties, as well as allowing subsequent surface modification reactions to take place.

In one embodiment, the drug impregnated coating includes at least one growth factor promoting agent, such as ReGeneraTing Agent (RGTA.

In another example, healing promoter 116A, 116B includes a plurality of loop-like structures, a plurality of tail-like structures, or both to promote to promote tissue incorporation, the formation of thrombosis, and fixation of an endoluminal stent graft, such as endoluminal stent graft 100, in the vessel. Loop structures exist in special PET material (velour Dacron). This Dacron material presents a non-regular/enterogenous surface topography that would dramatically enhanced cell/tissue adhesion. Alternatively, these loops or tail like structures would be manually braided into the original stent graft material. A particular diameter specification is not important. The loops (or tails for that matter) should provide for a "porous coating-like" structure of at least 1 µm, more preferably at least 5 µm, most preferably at least 10 µm thickness.

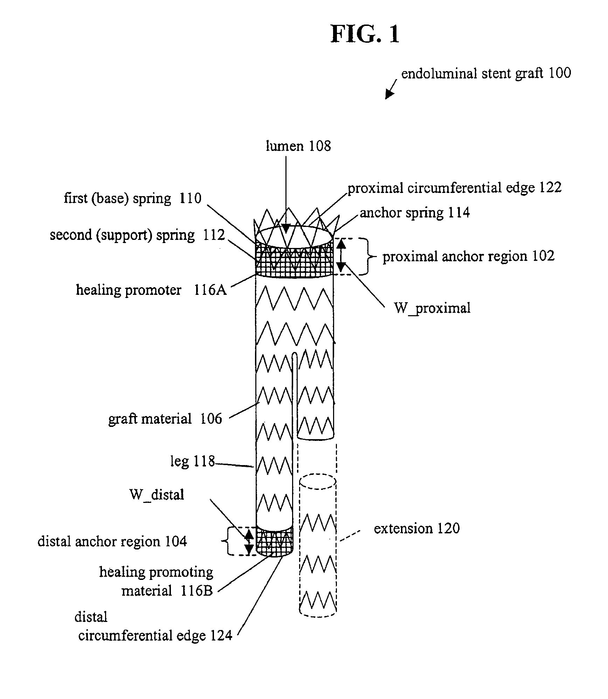

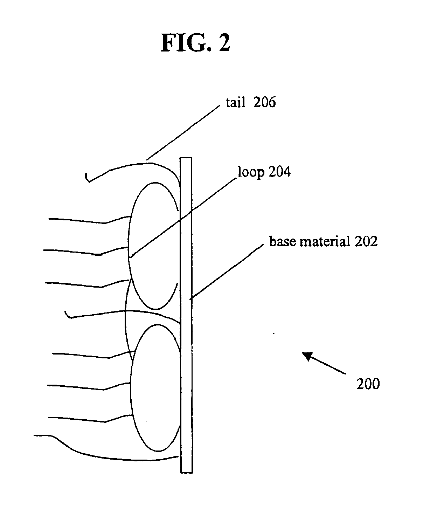

FIG. 2 illustrates one example of a healing promoter 116A, 116B including one or more loop-like structures 204, one or more tail-like structures 206, or a combination of one or more loop-like structures 204 and one or more tail-like structures 206. Referring now to FIGS. 1 and 2 together, healing promoter 116A, 116B includes a support material 202 having one or more loop-like structures 204, herein termed loops 204, and one or more tail-like structures 206, herein termed tails 206, attached.

In yet another example, loops 204, tails 206, or both are made of a biocompatible copolymer. For example, loops 204, tails 206, or both are made of polyester, such as Dacron or polytetrafluoroethylene (PTFE). Additional extensive list of polymers is provided earlier.

In one application, loops 204, tails 206, or both are attached by sewing or weaving. Loops 204, tails 206, or both are attached to graft material 106, the stent structure, such as any of first base spring 110, second support spring 112, and anchor spring 114, among others, or both to promote tissue incorporation and the fixation of a stent graft, such as stent graft 100, in a vessel. In one embodiment, loops 204, tails 206, or may both initiate swelling when in contact with blood for the first few seconds prior to deployment. For the most part these loops and tails will largely be in contact with the tissue wall and not with (flowing) blood. They might get in contact with interstitial fluid with time. Hydrophylic polymers will swell, such as collagen-derived loops or tails or hydrophilic polyurethanes.

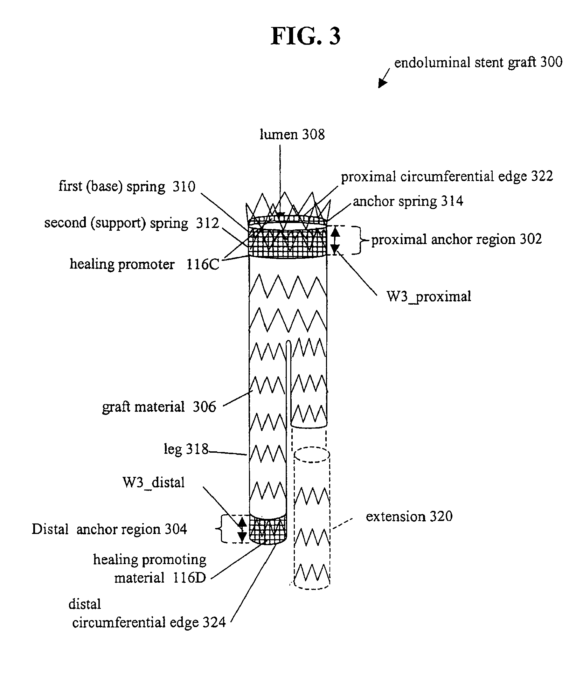

The various embodiments of the invention described herein with reference to FIG. 1, included healing promoter 116A, 116B attached to the exterior of stent graft 100, and in particular to the exterior circumferential side of graft material 106. In other embodiments in accordance with the invention, the healing promoter is additionally attached to a portion of an interior circumferential side of an endoluminal stent graft as further described herein with reference to FIG. 3.

FIG. 3 illustrates one example of an endoluminal stent graft 300 (308) including healing promoter 116C located within a proximal anchor region 302 on both an exterior and interior circumferential surface of stent graft 300, and healing promoter 116D within a distal anchor region 304. Endoluminal stent graft 300, herein termed simply stent graft 300, includes: a graft material 306 formed of a first material; healing promoter 116C, 116D positioned about an exterior circumferential surface and an interior circumferential surface of the first material,; and a stent structure of shaped springs, such as a first (base) spring 310, a second (support) spring 312, and an anchor spring 314, among others, distributed within stent graft 300 and attached to graft material 306. In the present embodiment, stent graft 300 is shaped to form a lumen 308 that bifurcates distally. Optionally, an extension 320 is included as part of stent graft 300.

In the present embodiment, graft material 306 is a material formed to limit the leakage of blood from lumen 308 through graft material 306. Examples of graft material 306 include substantially non-porous fabrics, such as low profile system (LPS) material, or densely knitted fabrics.

As illustrated, proximal anchor region 302 is located at a proximal neck of stent graft 300, and healing promoter 116C is within distal anchor region 302 on an exterior circumferential surface of graft material 306 and on an interior circumferential surface of graft material 306. Proximal anchor region 302 extends longitudinally from a proximal circumferential edge 322 longitudinally toward the distal end of stent graft 300 a specified distance W3_proximal. In one example, healing promoter 116C overlaps proximal circumferential edge 322 from the exterior side of graft material 306 to the interior side of graft material 306. In another example, healing promoter 116C is included on the interior circumferential surface of graft material 306 within distal anchor region 302, but has a length less than specified distance W3_proximal.

Distal anchor region 304 is located at the distal neck of leg 318 of stent graft 300, and healing promoter 116D is within distal anchor region 304 on the exterior circumferential surface of graft material 306 and on the interior circumferential side of graft material 306. In one example, distal anchor region 304 extends from a distal circumferential edge 324 longitudinally towards the proximal end of stent graft 300 a specified distance W3_distal. In one example, healing promoter 116D overlaps distal circumferential edge 324 from the exterior side of graft material 306 to the interior side of graft material 306. In another example, healing promoter 116D is included on an interior circumferential surface of graft material 306 within proximal anchor region 304, but has a length less than specified distance W3_proximal.

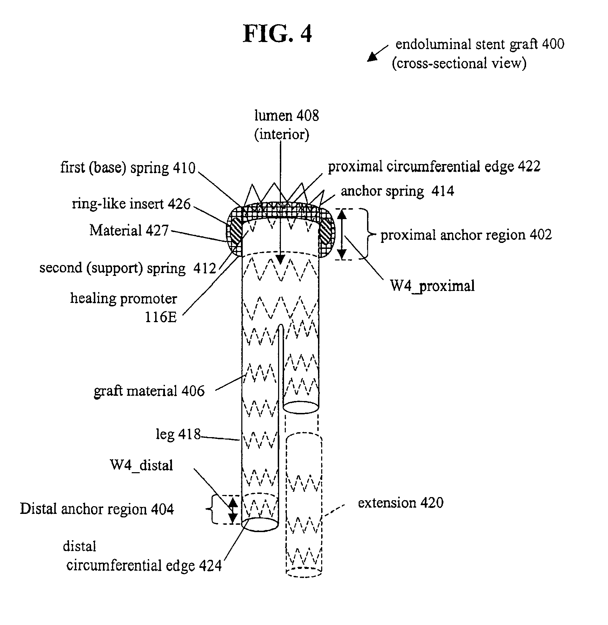

In some applications, it may be desirable to provide more intimate contact between an endoluminal stent graft and the interior vessel walls by including a ring-like insert within healing promoter 116E as further described herein with reference to FIG. 4. Due to the enhanced contact provided by the ring-like insert, inclusion of a material, e.g., a fabric or a coating, surrounding the ring-like insert of healing promoter 116E at the proximal anchor region is optional.

FIG. 4 illustrates one example of an endoluminal stent graft 400 including healing promoter 116E located within a proximal anchor region 402 and further including a ring-like insert 426. Endoluminal stent graft 400, herein termed simply stent graft 400, includes: a graft material 406 formed of a first material; healing promoter 116E including ring-like insert 426 that, in this is example is covered by a material 427; and a stent structure of shaped springs, such as a first (base) spring 410, a second (support) spring 412, and an anchor spring 414, among others, distributed within stent graft 400 and attached to graft material 406. In another embodiment, not shown, material 427 is not used and ring-like insert 426 is sewn on graft material 406.

In the present embodiment, stent graft 400 is shaped to form a lumen 408 that bifurcates. Optionally, an extension 420 is included as part of stent graft 400.

Proximal anchor region 402 extends from a proximal circumferential edge 422 longitudinally toward the distal end of stent graft 400 a specified distance W4_proximal. Ring-like insert 426 is included within proximal anchor region 402. In one example, material 427 covers ring-like insert 426 and overlaps proximal circumferential edge 422 from the exterior side of graft material 406, i.e., the non-luminal side, to the interior side of graft material 406, i.e., the luminal side. In some applications, healing promoter 116E (See Fig. 3) also is attached to the interior side of graft material 406 within distal anchor region 402, but has a length less than specified distance W4_proximal.

The thickness of ring-like insert 426 is selected to provide better intimate contact between stent graft 400 and a vessel in which stent graft 400 is positioned. For example, a ring like a ring as used in annuloplasty devices for repair of cardiac valves: 2-3mm in thickness. In one example, ring-like insert 426 is formed of silicon rubber. In another example, ring-like insert 426 is formed of one or more closed metal bands. In yet another example, ring-like insert 426 is formed of one or more open metal bands. Other polymers than silicone can be used, as long as they are as flexible

In one application, ring-like insert 426 is a reservoir of healing promoting agents and thus includes one or more healing promoting agent(s). The one or more healing promoting agent(s) are selected from a group consisting of growth factors, growth factor protecting agents, such as RGTA, alone or in combination with (heparin binding) growth factors. Examples of healing promoting growth factors are FGF, VEGF, PDGF, IGF, EGF). One could further think of pro- and anti-inflammatory cytokines as possible suitable healing promoting agents. Examples would be members of the interleukin family, TNFalpha, IFN, TGFbeta and others. Pseudo healing promoting agents that may be used include: hormone, antibiotics, immunosuppressant, and gene containing.
The metal inserts could be dip-coated thus being provided with a polymer overcoat from which the agent can be released. The polymer insert can be provided with bioactive agents in several ways: pre-mixing polymer with agents then forming the ring insert (will depend on agent stability throughout processing); solvent swelling-induced imbibement with agents (again will depend on agent stability throughout processing). Alternatively, both metal and polymer inserts can be provided with surface pits or grooves into which the agents can be deposited for subsequent release. In some applications, the one or more healing promoting agents are slow releasing up to maximally 28 days is preferred

To provide efficient folding and unfolding of stent graft 400 using a catheter-based delivery technique, ring-like insert 426 is cut in at one point to allow folding of ring-like insert 426 by overlapping the cut free ends of ring-like insert 426 with subsequent unfolding when positioned in a vessel lumen.

Although the example illustrated and described with reference to FIG. 4 is described as including ring-like insert 426 in proximal anchor region 402, but not distal anchor region 404. In other examples, a healing promoting material and a ring-like insert are included in both proximal anchor region 402 and distal anchor region 404. In still other example, healing promoting material and ring-like insert 426 are included in proximal anchor region 402, but only a healing promoting material is included in distal anchor region 404.

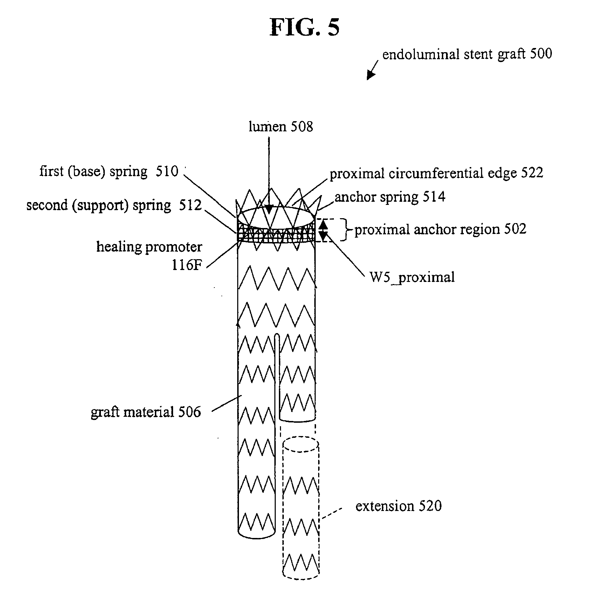

FIGS. 5 to 7 illustrate additional examples of an endoluminal stent graft in which a healing promoter is included in selected portions defined by the stent structure of the endoluminal stent graft.

In particular, FIG. 5 illustrates one example of an endoluminal stent graft 500 including healing promoter 116F located within a proximal anchor region 502. In FIG. 5, endoluminal stent graft 500, herein termed simply stent graft 500, includes: a graft material 506, i.e., a first material; healing promoter 116F positioned about an exterior circumferential surface of the first material; and a stent structure of shaped springs, such as a first (base) spring 510, a second (support) spring 512, and an anchor spring 514, among others, distributed within stent graft 500 and attached to graft material 506. Stent graft 500 is shaped to form a lumen 508 that bifurcates. Optionally, an extension 520 is included as part of stent graft 500.

Graft material 506 is a material that limits the leakage of blood from lumen 508 through graft material 506. Examples of graft material 506 include substantially non-porous fabrics, such as low profile system (LPS) material, or densely knitted fabrics.

As illustrated, proximal anchor region 502 is located at a proximal neck of stent graft 500, and healing promoter 116F is included within distal anchor region 502 on the exterior circumferential surface of graft material 506. Proximal anchor region 502 extends longitudinally from a proximal circumferential edge 522 toward the distal end of stent graft 500 a specified distance W5_proximal. Substantially all of first (base) spring 510 and nearly one-half (1/2) of second (support) spring 512 are sewn to healing promoter 116F and healing promoter 116F is sewn to graft material 506.

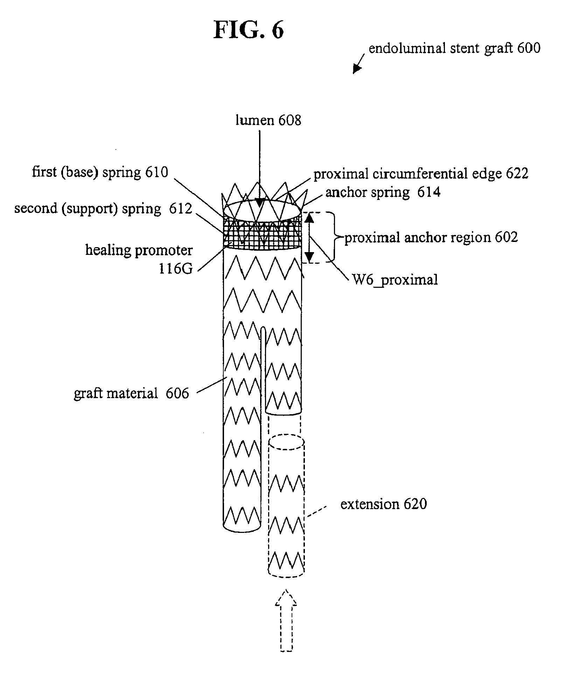

FIG. 6 illustrates one example of an endoluminal stent graft 600 including healing promoter 116G located within a proximal anchor region 602. Endoluminal stent graft 600, herein termed simply stent graft 600, includes: a graft material 606, i.e., a first material; healing promoter 116G positioned about an exterior circumferential surface of the first material; and a stent structure of shaped springs, such as a first (base) spring 610, a second (support) spring 612, and an anchor spring 614, among others, distributed within stent graft 600 and attached to graft material 606. Stent graft 600 is shaped to form a lumen 608 that bifurcates. Optionally, an extension 620 is included as part of stent graft 600.

Graft material 606 is a material that limits the leakage of blood from lumen 608 through graft material 606. Examples of graft material 606 include substantially non-porous fabrics, such as low profile system (LPS) material, or densely knitted fabrics.

As illustrated, proximal anchor region 602 is located at a proximal neck of stent graft 600, and healing promoter 116G is included within distal anchor region 602 on the exterior side of graft material 606. Proximal anchor region 602 extends longitudinally from a proximal circumferential edge 622 toward the distal end of stent graft 600 a specified distance W6_proximal. Substantially all of first (base) spring 610 and second (support) spring 612 are sewn to healing promoter 116G and healing promoter 116G is sewn to graft material 606.

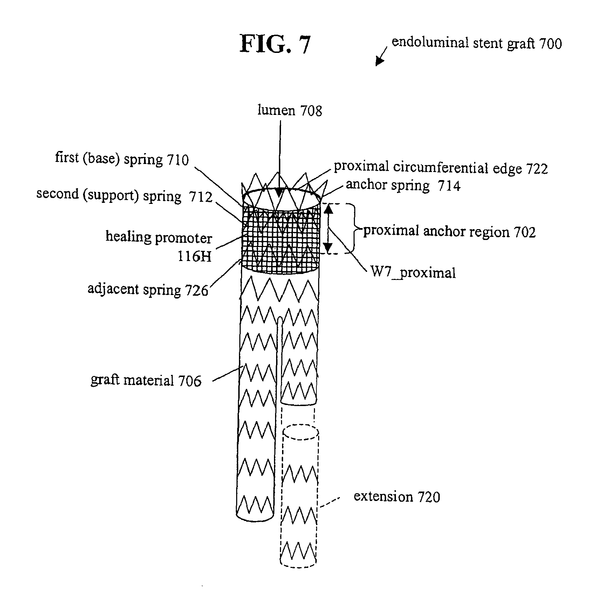

FIG. 7 illustrates one example of an endoluminal stent graft 700 including healing promoter 116H located within a proximal anchor region 702. Endoluminal stent graft 700, herein termed simply stent graft 700, includes: a graft material 706, i.e., a first material; healing promoter 116H positioned about an exterior circumferential surface of the first material; and a stent structure of shaped springs, such as a first (base) spring 710, a second (support) spring 712, and an anchor spring 714, among others, distributed within stent graft 700 and attached to graft material 706. Stent graft 700 is shaped to form a lumen 708 that bifurcates. Optionally, an extension 720 is included as part of stent graft 700.

Graft material 706 is a material that limits the leakage of blood from lumen 708 through graft material 706. Examples of graft material 706 include substantially non-porous fabrics, such as low profile system (LPS) material, or densely knitted fabrics.

As illustrated, proximal anchor region 702 is located at a proximal neck of stent graft 700, and healing promoter 116H is included within distal anchor region 702 on the exterior side of graft material 706, i.e., the non-luminal side. Proximal anchor region 702 extends longitudinally from a proximal circumferential edge 722 longitudinally toward the distal end of stent graft 700 a specified distance W7_proximal. Substantially all of first (base) spring 710 and second (support) spring 712, and a portion of a next spring adjacent second (support) spring 712, i.e., adjacent spring 726, are sewn to healing promoter 116H and healing promoter 116H is sewn to graft material 706.

Although the examples illustrated and described with reference to FIGS. 5-7 are illustrated as including the healing promoter within the proximal anchor region, in other embodiments, the healing promoter can further be included within the distal anchor region of a leg, and/or on a distal region of an extension to the endoluminal stent graft, for example, such as any of extensions 520, 620, and 720.

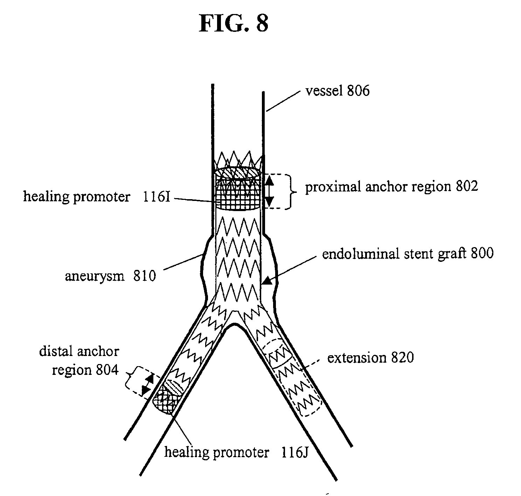

FIG. 8 illustrates one example of an endoluminal stent graft 800, including healing promoter 116I located within a proximal anchor region 802 and healing promoter 116J located with a distal anchor region 804, positioned within a vessel 806. Endoluminal stent graft 800 is positioned within vessel 806, for example, using a catheter-based delivery technique, such that healing promoter 116I contacts the wall of vessel 806 in proximal anchor region 802 and that healing promoter 116J contacts the wall of vessel 816 in proximal anchor region 804. Healing promoter 116I promotes cellular in growth from vessel 806 and consequent fixation of endoluminal stent graft 800 in vessel 806, thus reducing the risk of distal migration and occurrence of endoleaks that could otherwise form at the side of the proximal and distal necks and the consequent feeding of aneurysm 810.

This disclosure provides examples according to the present invention. In particular, examples having the healing promoter located in a proximal anchor region and in distal anchor regions can be varied to eliminate the healing promoter in the distal anchor regions. Further, examples having the healing promoter located in a proximal anchor region can be varied to further include the healing promoter in one or more distal anchor regions. Additionally, examples illustrated and described without ring-like insert 426, can be varied to further include ring-like insert 426. Also, the healing promoter can be of different materials, such as a fabric at a proximal anchor region and a coating in a distal anchor region. Numerous variations, whether explicitly provided for by the specification or implied by the specification or not, such as variations in structure, dimension, type of material and manufacturing process may be implemented by one of skill in the art in view of this disclosure.

Also, although the above examples illustrated and described herein used an endoluminal stent graft having a bifurcated structure, these examples are applicable to a wide variety of endoluminal stent graft designs, such as other bifurcated and non-bifurcated designs, as well as other stent structures, such as other spring structures, strut structures, and interlocking structures, among others.

## Claims

1. An endoluminal stent graft (100) comprising:
a stent structure;
a graft material (106) attached to said stent structure and having a circumferential edge (122, 124);
an anchor region (102, 104) extending longitudinally along said graft material (106) from said circumferential edge (122, 124); and
a healing promoter (116A, 116B) located within said anchor region (102, 104) wherein said healing promoter (116A, 116B) aids in anchoring said endoluminal stent graft (100) in a vessel, wherein said healing promoter (116A, 116B) includes a first portion on an exterior circumferential surface of said graft material (106).

2. The endoluminal stent graft of Claim 1, wherein said healing promoter (116A, 116B) includes a second portion on an interior circumferential surface of said graft material (106).

3. The endoluminal stent graft of Claim 1 or 2, wherein said healing promoter (116A, 116B) is attached to a portion of said graft material (106) by stitches.

4. The endoluminal stent graft of Claim 1 or 2, wherein said healing promoter (116A, 116B) is attached to a portion of said graft material (106) by an adhesive.

5. The endoluminal stent graft of any of Claims 1 to 4 wherein said healing promoter (116E) comprises:
a ring-like insert (426) mounted around an exterior circumferential surface of said graft material (406) in said anchor region (402).

6. The endoluminal stent graft of Claim 5, wherein said ring-like insert (426) is formed of silicon rubber.

7. The endoluminal stent graft of Claim 5, wherein said ring-like insert (426) is formed of one or more closed metal bands.

8. The endoluminal stent graft of Claim 5, wherein said ring-like insert (426) is formed of one or more open metal bands.

9. The endoluminal stent graft of Claim 5, wherein said ring-like (426) insert is cut in at one point to allow folding of the ring-like insert (426) by overlapping free ends of the ring-like insert.

10. The endoluminal stent graft of any of Claims 5 to 9, wherein said ring-like insert (426) includes at least one healing promoting agent.

11. The endoluminal stent graft of Claim 10, wherein the at least one healing promoting agent is selected from a group consisting of a growth factor, growth factor protecting agents, and pseudo healing promoting agents.

12. The endoluminal stent graft of any of Claims 1 to 4, wherein said endoluminal stent graft further comprises:
a first base spring (510) attached to said graft material (506);
a second support spring (512) attached to said graft material (506); and
a next spring (514) adjacent said second support spring (512) and attached to said graft material (506).

13. The endoluminal stent graft of Claim 12, wherein substantially all of said first base spring (510) and at least one-half of said second support spring (512) are sewn to said healing promoter (116F) and said healing promoter (116F) is sewn to said graft material (506) within a proximal anchor region (502) of said endoluminal stent graft (500).

14. The endoluminal stent graft of Claim 12, wherein substantially all of said first base spring (510) and substantially all of said second support spring (512) are sewn to said healing promoter (116F) and said healing promoter (116F) is sewn to said graft material (506) within a proximal anchor region (502) of said endoluminal stent graft (500).

15. The endoluminal stent graft of Claim 12, wherein substantially all of said first base spring (510) and said second support spring (512), and a portion of said next spring (514) adjacent said second support spring (512) are sewn to said healing promoter (116F), and said healing promoter (116F) is sewn to said graft material (506) within a distal anchor region (502) of said endoluminal stent graft (500).

16. The endoluminal stent graft of Claim 12, wherein substantially all of said first base spring (510) and said second support spring (512), and said next spring (514) adjacent said second support spring (512) are sewn to said healing promoter (116F), and said healing promoter (116F) is sewn to said graft material (506) within a proximal anchor region (502) of said endoluminal stent graft (500).

17. The endoluminal stent graft of any of the preceding Claims wherein said healing promoter comprises:
a porous fabric.

18. The endoluminal stent graft of Claim 17, wherein the porous fabric is a knitted Dacron fabric.

19. The endoluminal stent graft of Claim 17 or 18, wherein the porous fabric has a pore size larger than 100 µm.

20. The endoluminal stent graft of any of the preceding Claims, wherein said healing promoter comprises:
at least one coating.

21. The endoluminal stent graft of Claim 20 wherein said coating comprises a collagen coating.

22. The endoluminal stent graft of Claim 20 wherein said coating comprises a drug impregnated coating that promotes formation of thrombosis and tissue incorporation between the endoluminal stent graft and a vessel.

23. The endoluminal stent graft of Claim 22 wherein said drug impregnated coating comprises at least a drug impregnated polymer.

24. The endoluminal stent graft of Claim 22 or 23, wherein said drug impregnated polymer is selected from a group consisting of polyvinyl alcohol and polyethylene glycol.

25. The endoluminal stent graft of any of the preceding Claim, wherein said healing promoter comprises:
at least a healing promoting agent.

26. The endoluminal stent graft of Claim 25, wherein said healing promoting agent is selected from a group consisting of a growth factor, growth factor protecting agents, and pseudo healing promoting agents.

27. The endoluminal stent graft of any of the preceding Claims, wherein said healing promoter comprises at least one loop-like (204) or tail like (206) structure that include at least one drug.

28. The endoluminal stent graft of Claim 27, wherein said at least one drug is a drug clotting factor.

29. The endoluminal stent graft of Claim 27, wherein said at least one drug is a drug tissue attachment factor.

30. The endoluminal stent graft of Claim 29, wherein said drug tissue attachment factor is slow releasing.
